# EUROPÄISCHE PATENTSCHRIFT

(11) **EP 1 140 757 B1**
(45) Veröffentlichungstag und Bekanntmachung des Hinweises auf die Patenterteilung: **23.07.2003**
(21) Anmeldenummer: 99964534.4
(22) Anmeldetag: 09.12.1999
(51) Int. Cl.: C07C 39/235, C07C 37/64, B01J 31/02

(54) **FLÜSSIGE FORMULIERUNG VON TETRABUTYLAMMONIUMPHENOLAT**
LIQUID TETRABUTYL AMMONIUM PHENOLATE FORMULATION
FORMULATION LIQUIDE DE PHENOLATE D'ALUMINIUM DE TETRABUTYLE

(30) Priorität: 21.12.1998 DE 19858967
(43) Veröffentlichungstag der Anmeldung: 10.10.2001
(73) Patentinhaber: Bayer Aktiengesellschaft, 51368 Leverkusen (DE)
(72) Erfinder: HESSE, Carsten, D-47918 Tönisvorst (DE); JANSEN, Ursula, D-47799 Krefeld (DE); RECHNER, Johann, D-47906 Kempen (DE)
(86) Internationale Anmeldenummer: EP9909689
(87) Internationale Veröffentlichungsnummer: WO00037402

(56) Entgegenhaltungen:
- EP-A- 0 362 854
- MAGONSKI J ET AL: "DISSOCIATION CONSTANTS OF SUBSTITUTED PHENOLS AND HOMOCONJUGATION CONSTANTS OF THE CORRESPONDING PHENOL-PHENOLATE SYSTEMS IN ACETONITRILE" JOURNAL OF THE CHEMICAL SOCIETY. FARADAY TRANSACTIONS,GB,ROYAL SOCIETY OF CHEMISTRY, CAMBRIDGE, Bd. 89, Nr. 1, 1993, Seiten 119-122, XP000867637 ISSN: 0956-5000 in der Anmeldung erwähnt

## Beschreibung

Die vorliegende Erfindung betrifft eine bei Raumtemperatur flüssige Formulierung von Tetrabutylammoniumphenolat und Verfahren zu ihrer Herstellung.

Tetraalkylammoniumphenolate sind bereits verschiedentlich bekannt geworden. So offenbaren J. Am. Chem. Soc. 103 (1983) 475 und Inorg. Chem. 24 (1985) 3465 die Herstellung von Tetraethylammoniumphenolat, aus DE-OS 22 03 448 ist die Darstellung von Tetrabutylammoniumphenolat bekannt, EP-A 244 799 lehrt die Herstellung eines Tetraalkylammoniumphenolate enthaltenden Elektrolyten.

Auch verschiedene Phenoladdukte von Tetraalkylammoniumphenolaten sind bereits bekannt geworden. J. Chem. Soc. Faraday Trans. 89 (1993) 119 offenbart die Herstellung von (Mono-)Phenoladdukten verschiedener Tetraalkylammoniumphenolate und aus EP-A 362 854 geht die Herstellung des Di(p-tert.-butylphenol)-Addukts von Tetrabutylammonium(p-tert.-butylphenolat) hervor.

Bei all diesen Verbindungen handelt es sich um bei Raumtemperatur feste Substanzen. Für eine großtechnische Anwendung ist es jedoch wünschenswert, eine bei Raumtemperatur flüssige Formulierung zur Verfügung zu haben, da dies die Dosierung stark vereinfacht.

Gegenstand der Erfindung sind Tetrabutylammoniumphenolat (TBAP) und Phenol im Gewichtsverhältnis 40:60 bis 70:30, bevorzugt 50:50 bis 60:40 enthaltende Formulierungen mit einem Erstarrungspunkt <25°C, bevorzugt <20°C.

Gegenstand der Erfindung sind weiterhin Verfahren zur Herstellung der erfindungsgemäßen Formulierungen. Die erfindungsgemäßen Formulierungen können hergestellt werden, indem man zunächst Natriumphenolat und Tetrabutylammoniumbromid (TBAB) separat bei Temperaturen >40°C in Phenol löst. Beim Zusammengeben der beiden Phasen fällt sofort Natriumbromid als feiner Niederschlag aus. Man destilliert unter vermindertem Druck überschüssiges Phenol ab, bis das gewünschte Mischungsverhältnis von TBAP und Phenol erreicht ist.

Nach Abkühlen auf Raumtemperatur wird aus der trüben Flüssigkeit Natriumbromid abgetrennt. Dies kann durch die üblichen, dem Fachmann bekannten Methoden geschehen, z.B. durch Filtrieren, Sedimentieren oder Zentrifugieren. Bevorzugt erfolgt die Abtrennung des Niederschlags durch Filtration. In einer besonders bevorzugten Ausführungsform wird die Filtration über eine Drucknutsche mit einer Filtermatte für feindisperse Materialien durchgeführt (Tiefenfiltration). Dabei ist der Natriumgehalt der filtrierten Lösung umso geringer, je feiner das verwendete Filter ist. Werden Filtermatten mit Rückhalteraten <40 µm verwendet, lassen sich Formulierungen erhalten, die weniger als 1000 ppm Natriumionen enthalten.

Da Natriumphenolat in Phenol eine verhältnismäßig geringe Löslichkeit aufweist, muß zunächst ein großer Überschuß an Phenol eingesetzt werden, das später wieder entfernt werden muß. In einer bevorzugten Ausführungsform des erfindungsgemäßen Verfahrens wird daher Natriumphenolat in wenig Wasser gelöst und zu einer phenolischen Lösung von Tetrabutylammoniumbromid gegeben. Das Wasser wird anschließend als Azeotrop mit Phenol destillativ entfernt. Bei dieser Variante ist zur Herstellung der erfindungsgemäßen Formulierungen nur etwa ein Zehntel der Menge Phenol erforderlich, die für die Herstellung über phenolische Natriumphenolatlösungen benötigt wird. Die Natriumgehalte der erhaltenen Lösungen liegen zudem deutlich niedriger, bei Werten <500 ppm.

Die erfindungsgemäßen Formulierungen werden bevorzugt als Bestandteil von Katalysatorsystemen eingesetzt, beispielsweise für die Produktion von Phenolharzen. Gegenstand der Erfindung ist daher auch die Verwendung der erfindungsgemäßen Formulierungen als Katalysatorbestandteil.

### Beispiele

Es wurden Salze mit einem Wassergehalt <500ppm eingesetzt. Die Bestimmung des Wassergehalts der Produkte erfolgte mit Karl-Fischer-Titration, der Bromidgehalt wurde argentometrisch bestimmt, der Natriumgehalt durch ICP.

### Beispiel 1

In einem 250 ml Zweihalskolben mit Vigreuxkolonne und Destillationsbrücke, Magnetrührfisch und Glasstopfen versetzte man 36,8 g (0,39 mol) geschmolzenes Phenol mit 36,8 g (0,11 mol) TBAB und ließ auf Raumtemperatur abkühlen (Lösung A). In einem 250 ml Becherglas löste man 19,4 g (0,11 mol) Natriumphenolat Trihydrat in 30 ml Wasser (Lösung B).

Anschließend tropfte man unter schnellem Rühren bei 60°C Lösung A zu B innerhalb von 15 min. zu. Zum Schluß versetzte man mit 43 g geschmolzenen Phenol. Von diesem Gemisch wurden im Vakuum ca. 64 g abdestilliert. Der Rückstand bestand aus einer dunkelbraunen Flüssigkeit mit einem feinen beigefarbenen Bodensatz, der über eine Drucknutsche, ausgelegt mit einer Filtermatte mit einer Rückhalterate von 15-35 µm (T2100, Seitz Filterwerke GmbH, D-55543 Bad Kreuznach) abfiltriert wurde.

Der Natrium-Gehalt betrug 250 ppm, der Bromidgehalt 0,21 Gew.-%.

### Beispiel 2

Die Durchführung erfolgte analog Beispiel 1, jedoch wurde zur Filtration eine Filtermatte mit einer Rückhalterate von 25-70 µm (T5500, Seitz Filterwerke GmbH, D-55543 Bad Kreuznach) eingesetzt.

Der Natrium-Gehalt betrug 1600 ppm, der Bromidgehalt 0,38 Gew.-%.

### Beispiel 3

In einem 1 1 Dreihalskolben löste man 19,4 g (0,11 mol) Natriumphenolat-Trihydrat in 600 g (6,4 mol) Phenol (Lösung A). In einem 100 ml Becherglas löste man in der Wärme 36,75 g (0,11 mol) TBAB in 36,75 g (0,39 mol) Phenol und ließ anschließend auf Raumtemperatur abkühlen (= Lösung B).

Man gab Lösung B zu Lösung A und destillierte bei ca. 60°C Innentemperatur im Vakuum überschüssiges Phenol ab, bis ein molares Verhältnis TBAP : PhOH von etwa 1 : 8 erreicht war. Man filtrierte vom ausgefallenen Natriumbromid über eine Drucknutsche mit einer Filtermatte mit einer Rückhalterate von 15-35 µm (T2100, Seitz Filterwerke GmbH, D-55543 Bad Kreuznach) ab.

Der Natrium-Gehalt betrug 700 ppm, der Bromidgehalt 0,21 Gew.-%.

### Beispiel 4

In einem 1 Dreihalskolben löste man 19,4 g (0,11 mol) Natriumphenolat Trihydrat in 30 ml Wasser (Lösung A). In einem 100 ml Becherglas löste man in der Wärme 36,75 g (0,11 mol) TBAB in 36,75 g (0,39 mol) Phenol und ließ anschließend auf Raumtemperatur abkühlen (= Lösung B).

Man gab Lösung B zu Lösung A und destillierte bei ca. 60°C Innentemperatur im Vakuum Wasser und überschüssiges Phenol ab, bis ein Gewichtsverhältnis von TBAP zu Phenol von etwa 1 : 1 erreicht war. Man filtrierte vom ausgefallenen Natriumbromid über eine Drucknutsche mit einer Filtermatte mit einer Rückhalterate von 15-35 µm (T2100, Seitz Filterwerke GmbH, D-55543 Bad Kreuznach) ab.

Der Natrium-Gehalt betrug 250 ppm, der Bromidgehalt 0,21 Gew.-%, der Wassergehalt 800 ppm.

### Beispiel 5

Beispiel 4 wurde wiederholt, jedoch wurde die Reaktionsmischung vor der Filtration noch für 16 Stunden bei 60°C gerührt.

Der Natrium-Gehalt des Filtrats betrug 180 ppm, der Bromidgehalt 0,18 Gew.-%, der Wassergehalt 900 ppm.

## Patentansprüche

1. Tetrabutylammoniumphenolat und Phenol im Gewichtsverhältnis 40:60 bis 70:30 enthaltende Formulierungen mit einem Erstarrungspunkt <25°C.

2. Verfahren zur Herstellung der Formulierungen gemäß Anspruch 1, bei dem phenolische Lösungen von Tetrabutylammoniumbromid und Natriumphenolat vereinigt werden, aus der Mischung überschüssiges Phenol abdestilliert und anschließend Natriumbromid abfiltriert wird.

3. Verfahren zur Herstellung der Formulierungen gemäß Anspruch 1, bei dem eine phenolische Lösung von Tetrabutylammoniumbromid und eine wäßrige Lösung von Natriumphenolat vereinigt werden, aus der Mischung Wasser und überschüssiges Phenol abdestilliert werden und anschließend Natriumbromid abfiltriert wird.

4. Verfahren zur Herstellung der Formulierungen gemäß Anspruch 1, bei dem phenolische Lösungen von Tetrabutylammoniumbromid und Natriumphenolat-Trihydrat vereinigt werden, aus der Mischung Wasser und überschüssiges Phenol abdestilliert und anschließend Natriumbromid abfiltriert wird.

5. Verwendung von Formulierungen gemäß Anspruch 1 als Katalysatorbestandteil.

## Claims

1. Formulations containing tetrabutylammonium phenolate and phenol in a weight ratio of from 40:60 to 70:30 and having a solidification point of <25°C.

2. Process for the preparation of the formulations according to claim 1, in which solutions of tetrabutylammonium bromide and sodium phenolate in phenol are combined, excess phenol is removed from the mixture by distillation, and sodium bromide is then filtered off.

3. Process for the preparation of the formulations according to claim 1, in which a solution of tetrabutylammonium bromide in phenol and an aqueous solution of sodium phenolate are combined, water and excess phenol are removed from the mixture by distillation, and sodium bromide is then filtered off.

4. Process for the preparation of the formulations according to claim 1, in which solutions of tetrabutylammonium bromide and sodium phenolate trihydrate in phenol are combined, water and excess phenol are removed from the mixture by distillation, and sodium bromide is then filtered off.

5. Use of formulations according to claim 1 as a catalyst constituent.

## Revendications

1. Formulations contenant du phénolate de tétrabutylammonium et du phénol dans un rapport pondéral 40:60 à 70:30 présentant un point de solidification < 25°C.

2. Procédé de préparation des formulations selon la revendication 1, dans lequel des solutions phénoliques de bromure de tétrabutylammonium et de phénolate de sodium sont réunies, le phénol en excès est séparé du mélange par distillation, puis le bromure de sodium est séparé par filtrage.

3. Procédé de préparation des formulations selon la revendication 1, dans lequel une solution phénoligue de bromure de tétrabutylammonium et une solution aqueuse de phénolate de sodium sont réunies, l'eau et le phénol en excès sont séparés du mélange par distillation, puis le bromure de sodium est séparé par filtrage.

4. Procédé de préparation des formulations selon la revendication 1, dans lequel les solutions phénoliques de bromure de tétrabutylammonium et de phénolate de sodium trihydrate sont réunies, l'eau et le phénol en excès sont séparés du mélange par distillation, puis le bromure de sodium est séparé par filtrage.

5. Utilisation des formulations selon la revendication 1 comme constituant de catalyseur.
